# EUROPEAN PATENT SPECIFICATION

(11) **EP 2 455 073 B1**
(45) Date of publication and mention of the grant of the patent: **30.09.2015**
(21) Application number: 11009130.3
(22) Date of filing: 17.11.2011
(51) Int. Cl.: A61K 31/01, A61K 31/722, A61K 31/723, A61K 36/062, A61P 3/06, A61K 31/366, A61K 36/81

(54) **Combination of substances for the treatment of patients with hypercholesterolemia and related disorders**
Kombination von Verbindungen zur Behandlung von Patienten mit Hypercholesterinämie und verwandten Erkrankungen
Combinaison de substances pour le traitement de patients souffrant d' hypercholestérolémie et des troubles lapparentés

(30) Priority: 18.11.2010 IT BO20100688
(43) Date of publication of application: 23.05.2012
(73) Proprietor: MEDIBASE S.R.L., 59100 Prato (IT)
(72) Inventor: Mangiapane, Andrea, 59021 Vaiano (PO) (IT)
(74) Representative: Mincone, Antimo

(56) References cited:
- WO-A1-00/04086
- KR-A- 20030 061 740
- Medibase: "Lesstat", , XP002640743, Retrieved from the Internet: URL:http://www.medibase.it/scheda.asp?codi ce=LESSTAT&lingua=ita [retrieved on 2011-06-06]
- BOKURA H ET AL: "Chitosan decreases total cholesterol in women: A randomized, double-blind, placebo-controlled trial", EUROPEAN JOURNAL OF CLINICAL NUTRITION, XX, XX, vol. 57, no. 5, 1 May 2003 (2003-05-01), pages 721-725, XP009090784,

## Description

The present invention relates to a composition or combination of substances usable for the treatment of patients with hypercholesterolemic syndrome and related disorders such as, for example, cardiovascular diseases and/or altered lipid metabolism, as well as for the prevention of such diseases.

In particular, the invention concerns a combination of Xanthan Gum, Chitosan, Lycopene, Monascus purpureus.

This combination provides surprisingly positive effects due to the synergy between the different substances contained in it.

The Xanthan Gum and Chitosan, in fact, decrease the absorption of dietary cholesterol, according to different actions.

The Xanthan Gum is an anionic polysaccharide used as a food additive and rheology modifier. It can be obtained by fermentation in pure culture of a carbohydrate (glucose or sucrose) by natural strains of the bacterium *Xanthomonas campestris,* purified by extraction with ethanol or propan-2-ol, dried and milled. It contains, as the main hexoses, D-glucose and D-mannose, and D-glucuronic acid and Pyruvic acid and it is prepared as salts of sodium, potassium or calcium.

Chitosan is a cationic linear polysaccharide composed of D-glucosamine and N-acetyl-D-glucosamine linked by β (1-4) bonds. Besides being used as a component of some detergents formulations, such as a component of shampoo, Chitosan is also used in energy-restricted diets for weight reduction, since it has the ability to bind dietary fat by preventing the absorption of the same.

It is known the combined use of Chitosan and Xanthan for supporting enzymes and drugs. In US-5620706 and US-5648252 are described polyaddition products including Chitosan and Xanthan, as well as in WO-00/04086, which limit the thermal and photochemical degradation of dietary or dermatological active principles supported by them. Furthermore, published papers in international journals demonstrate the ability of Chitosan-Xanthan complex to constitute a transport means for the controlled release of drugs, with a capacity of microencapsulation of active ingredients. Such as the paper "The Utilization of Chitosan-xanthan Gum to Prolong Drug release from capsules"- Department of Pharmaceutical Technology Faculty of Pharmacy, Silpakorn University, Nakorn, Thailand. Also U.S. Patent Application 20080260818 accurately describes the use of these substances to determine a controlled absorption of synthetic statins in the intestine. In this case it is demonstrated an increase in the bioavailability of the active ingredient that allows a significant increase in plasma level of active ingredients, prolonging the period of activity and, at the same time, decreasing the severity of potential side effects thereof.

Korean patent application KR20030039654 discloses a beverage containing monascus purpureus and antioxidants including lycopene, for use in reducing cholesterol levels.

None of the documents of the prior art describes the use of Chitosan and Xanthan in combination with a carotenoid and a yeast, not being known or expected possible implications regarding the benefit / side effects. In particular, WO-00/04086 relates to hydrogel for the release of vitamins, and U.S. Patent Application 20080260818 relates to the release of synthetic statins.

Lycopene is a non-polar alkyl compound made of only hydrogen and carbon, in the group of carotenoids. It can also be used as a food additive. Carotenoids, in general, are effective antioxidants due to their effectiveness as a "scavenger" (literally scavenging) of free radicals. Among the carotenoids, lycopene appears to be the most efficient "oxygen quencher", thanks to the presence of two additional double bonds compared to the structure of other carotenoids.

The combination of the present invention uses a particular type of lycopene from tomato extract by using a matrix of milk proteins. This preparation has been developed by the Nestle company and the above mentioned physical process significantly increases the bioavailability. Lycopene on the milk protein, acts as a real increase in plasma levels of lycopene itself, from about 0.26 micromoles / L (baseline) to about 0.52 to 0.96 micromol / L (after treatment). This increase is able to reduce the oxidation of lipoproteins up to 80%, as reported in several works. In fact it is proved that the oxidation of LDL is one of the main contributory causes of the worst records of atherosclerosis and thrombotic gaps that cause the aggravation of cardiovascular fact, as demonstrated by the literature that shows that 50% of patients suffering a myocardial infarction event have no abnormalities of cholesterol. The Monascus Purpureus is a yeast contained in the fermented red rice, a traditional component of nutrition and herbal medicine Chinese for centuries, already mentioned in the Pharmacopoeia "BenCaoGangMu-DanShiBuYi", published at the time of the Ming Dynasty (1368 - 1644).

In the late 90's several studies have shown the efficacy of Monascus Rice Purpureus extract especially due to the presence among its components of a HMG-CoA reductase inhibitors (monacolin K), liver enzyme responsible for producing cholesterol, which inhibiting the action of this enzyme was able to reduce the hepatic synthesis of cholesterol.

The contribution that the Monascus Purpureus provides to the synergistic action of the components of the combination of the invention, determines the decreased synthesis of cholesterol with a statin-like mechanism and, therefore, with a block of the mevalonate pathway.

Studies conducted by the Applicant of this patent application have led to affirm that the administration of the association of the invention results in a significant reduction in total cholesterol and LDL as well as an increase in the values of HDL cholesterol and a decrease in the values of triglyceridemia.

Advantageously, the simultaneous use of Xanthan gum (anionic polysaccharide) and Chitosan (cationic polysaccharide) gives rise to a polyaddition product crosslinked in the stomach (acidic pH). The gelation obtained, the so-called ionotropic, becomes reversible during the intestinal transit (basic pH) determining the controlled release of active ingredients (monacolin K) trapped within the lattice structure, thus increasing the time of absorption, bioavailability, and consequently an increase in the percentage of reduction of cholesterol levels, compared to the use of red rice extract isolated as commonly happens in all the products on the market. The association produces a clear advantage to use. Also in this case there is also the decrease of side effects attributable to the use of the extract of red rice (increased blood levels of liver transaminases and CK muscle).

It is important to highlight that the association is a natural fulfillment of action only through a significant reduction of LDL oxidation obtained for the presence of milk proteins adsorbed on Lycopene. In this way, only this association (Chitosan-Xanthan-Lycopene-red rice extract) is able to intervene effectively and simultaneously on all the main causes of cardiovascular pejorative situation in these patients.

The first statistical evaluations of use of the combination, show decreases of the total cholesterol by 20-25%, approaching a lot in to the culls guaranteed by the synthetic statins, but not presenting its side-effects. On the other hand, the use of red rice extract at the doses allowed by law (3mg/die), provides rebates for "only" 10-12% corroborating thus improving the therapeutic response of the extract red rice, only if used in combination with the polysaccharide components of the present invention. It is clear that the combination of elements that form the association provides a result not expected a priori and not indicated by previous studies in this area. In fact, although being known for many years the properties of Monascus Purpureus and the characteristics of Chitosan and Xanthan, no one had so far combined with lycopene, to provide a single product that can provide a reduction of total cholesterol which, in studies conducted so far by the Applicant, was about 25%; in other words, the association of the invention will definitely get results comparable to those provided by synthetic statins, without resulting in adverse side effects.

In practice, the use of this association is particularly beneficial as an adjunct in the treatment and prevention of cardiovascular diseases, due to the activities described earlier in the treatment of patients with abnormal lipid metabolism (elevated blood levels of cholesterol).

A preferred but not limiting formulation of the association may be the following for a capsule/tablet that weighs 1250mg:
Xanthan gum: 400 mg;
Chitosan tit. 90%: 100 mg;
Lycopene tit.6%: 63 mg;

Monascus purpureus tit. 1.5%: 200 mg (equivalent to 3 mg of monacolin K). In terms of percentage by weight of each tablet, the composition, preferably but not limited in its formulation, can be expressed as follows: about 32% xanthan gum, chitosan about 8%, about 0-28% lycopene, Monascus purpureus about 5,08%.

In addition to these components, there are also a number of ingredients of various kinds, which may vary and are only used for the forming, pressing and tablet coating. As an example, these components may be: Magnesium stearate, gum arabic, sucrose, silicon dioxide, glycerol Microcrystalline Cellulose, Hydroxypropyl cellulose, etc.... The quantity of the components listed above can vary independently of each other, in the intervals set out below:
Xanthan gum: between 10 and 2000 mg;
Chitosan: between 10 and 1000 mg;
Lycopene 6% between 1 and 1000 mg;
Monascus purpureus tit. 1.5%: between 1 and 1000 mg.

With regard to the properties of individual components we can talk about synergy as each component operates through a different mechanism on the hypercholesterolemic problem; in fact, as stated above:
- Xanthan gum and Chitosan decrease the absorption of dietary cholesterol and increase the bioavailability and facilitate a controlled release of active ingredient of Monascus purpureus;
- Lycopene intervenes with its strong antioxidant capacity on the reduction of LDL oxidation, which is a major cause of atherosclerosis and that cause detachment of thrombotic which cause cardiovascular aggravation;
- the extract of Monascus Purpureus decreases the synthesis of cholesterol by a mechanism statine-similar and then with a block of the mevalonate pathway.

So the result, not obtainable with the products on the market until now, is threefold: reduction of the absorption, decrease of synthesis, decrease of oxidative risk and, in addition, as stated above, an increase of bioavailability and plasma / time level of active ingredient of Monascus purpureus.

## Claims

1. Combination of substances for use in the treatment of patients with hypercholesterolemia or of patients with hypercholesterolemic syndrome, cardiovascular diseases and/or altered lipid metabolism, as well as for the prevention of such diseases **characterized in that** comprises: Xanthan gum, Chitosan, Lycopene and Monascus purpureus.

2. Combination for use according to claim 1, **characterized in that** the Chitosan is at a titration of 90%.

3. Combination for use according to claim 1, **characterized in that** Lycopene is at a titration of 6% or greater.

4. Combination for use according to claim 1, **characterized in that** the Monascus purpureus is at a titration of 1.5% or greater.

5. Combination of substances for use in the treatment of patients with hypercholesterolemia or of patients with hypercholesterolemic syndrome, cardiovascular diseases and/or altered lipid metabolism, as well as for the prevention of such diseases **characterized in that** comprises, per tablet of 1250 mg, an amount of Xanthan gum of 400 mg, an amount of Chitosan at 90% of 100 mg, an amount of Lycopene at 6% of 63 mg, an amount of Monascus purpureus at 1.5% of 200 mg, corresponding to 3.0 mg of monacolin K.

## Patentansprüche

1. Zusammensetzung von Stoffen zur Behandlung von Patienten mit Hypercholesterinämie oder von Patienten mit Hypercholesterinämie-Syndrom, Herz- und/oder Kreislaufstörungen und / oder verändertem Lipidstoffwechsel sowie zur Prävention dieser Störungen, **dadurch gekennzeichnet, dass** sie Xanthan Gum, Chitosan, Lycopin und Monascus purpureus enthält.

2. Zusammensetzung zur Verwendung nach Anspruch 1, enthaltend Chitosan bei einer Titration von 90%.

3. Zusammensetzung zur Verwendung nach Anspruch 1, enthaltend Lycopin bei einer Titration von 6% oder mehr.

4. Zusammensetzung zur Verwendung nach Anspruch 1, enthaltend Monascus purpureus bei einer Titration von 1,5% oder mehr.

5. Zusammensetzung von Stoffen zur Behandlung von Patienten mit Hypercholesterinämie oder von Patienten mit Hypercholesterinämie-Syndrom, Herz- und/oder Kreislaufstörungen und / oder verändertem Lipidstoffwechsel sowie zur Prävention dieser Störungen, **dadurch gekennzeichnet, dass** sie Xanthangummi in Mengen von 400 mg, Chitosan mit 90% Trit. in Mengen von 100 mg, Lycopin 6% in Mengen von 63 mg, Monascus purpureus 1,5% in Mengen von 200 mg, entsprechend 3,0 mg Monacolin K, pro Tablette von 1250 mg enthält.

## Revendications

1. Combinaison de substances pour usage dans le traitement de patients atteints d'hypercholestérolémie ou de patients atteints du syndrome hypercholestérolémique, les maladies cardiovasculaires et / ou le métabolisme des lipides modifiés, ainsi que pour la prévention de ces maladies, **caractérisé en ce que** comprend: la gomme xanthane, le chitosane, le lycopène et Monascus purpureus.

2. Combinaison pour usage selon la revendication 1, **caractérisé en ce que** le chitosane est à un titrage de 90%.

3. Combinaison pour usage selon la revendication 1, **caractérisé en ce que** le lycopène est à un titrage de 6% ou plus.

4. Combinaison pour usage selon la revendication 1, **caractérisé en ce que** la Monascus purpureus est à un titrage de 1,5% ou plus.

5. Combinaison de substances pour usage dans le traitement des patients atteints d'hypercholestérolémie ou de patients atteints du syndrome hypercholestérolémique, les maladies cardiovasculaires et / ou le métabolisme des lipides modifiés, ainsi que pour la prévention de ces maladies, **caractérisé en ce qu'**il comprend, par une tablette de 1250 mg, une quantité de gomme xanthane de 400 mg, une quantité de chitosan à 90% de 100 mg, une quantité de lycopène à 6% de 63 mg, une quantité de Monascus purpureus à 1,5% de 200 mg, ce qui correspond à 3,0 mg de monacoline K.
